# EUROPEAN PATENT APPLICATION

(11) **EP 4 115 832 A1**
(43) Date of publication of application: **11.01.2023**
(21) Application number: 22155820.8
(22) Date of filing: 09.02.2022
(51) Int. Cl.: A61B 18/14, A61B 18/00

(54) **PULSED FIELD ABLATION CATHETER**

(30) Priority: 09.07.2021 US 202163220312 P; 07.01.2022 US 202217570924
(71) Applicant: Biosense Webster (Israel) Ltd., Yokneam 2066717 (IL)
(72) Inventor: RAO, Anand, Irvine, 92618 (US); SELKEE, Thomas, Irvine, 92618 (US); DATTA, Keshava, Irvine, 92618 (US); NGUYEN, Thanh, Irvine, 92618 (US)
(74) Representative: Carpmaels & Ransford LLP

(57) **Abstract**

A catheter can have a distal circular region that can contract in circumference via manipulation of a pull wire. The circular region can have electrodes distributed around the circumference that are suitable for mapping and/or ablation, and preferably suitable for IRE ablation. The catheter can include a cross-over region near a distal end of a shaft in which elongated elements extend at an angle to the longitudinal axis. The cross-over region can be bounded by an intermediate tube having four lumens and a distal tube having three lumens. The circular region can include structural features to facilitate contraction such as a support member having a preferable bending direction, polymer tubing segments positioned to inhibit bending stress on electrodes, navigation sensors positioned to inhibit bending stress on said sensors and electrodes, and a distal assembly at a distal end of the circular region.

## Description

### FIELD

The present invention relates to a catheter that is particularly useful for performing pulsed field ablation within or near a heart. The catheter may also be useful for mapping and/or thermal ablation using radio frequency electrical signals.

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Sources of undesired signals are typically located in tissue of the atria a ventricle. Regardless of source, unwanted signals are conducted elsewhere through heart tissue where they can initiate or continue arrhythmia.

Treatment of cardiac arrhythmia can include disrupting the conducting pathway of electrical signals causing arrhythmia to cease or modify the propagation of unwanted electrical signals from one portion of the heart to another. Such procedures typically include a two-step process: (1) mapping; and (2) ablation. During mapping, a catheter having an end effector having preferably a high density of electrodes is moved across target tissue, electrical signals are acquired from each electrode, and a map is generated based on the acquired signals. During ablation, non-conducting lesions are formed at regions selected based on the map to disrupt electrical signals through those regions. Presently the most common ablation technique involves applying radio frequency (RF) electrical signals via electrodes to tissue to generate heat. Irreversible electroporation (IRE) ablation is a more recently developed technique which involves applying short duration high voltage pulses across tissue to cause cell death.

Differing objectives of mapping, ablation with RF signals, and IRE ablation generally result in differing catheter design goals. As a non-exhaustive list, some catheters having a circular, semicircular, or helical end effector for mapping and/or ablation are described in U.S. Patent No. 6,973,339, U.S. Patent No. 7,371,232, U.S. Patent No. 8,275,440, U.S. Patent No. 8,475,450, U.S. Patent No. 8,600,472, U.S. Patent No. 8,608,735, U.S. Patent No. 9,050,010, U.S. Patent No. 9,788,893, U.S. Patent No. 9,848,948, and U.S. Patent Pub. No. 2017/0100188, each of which are incorporated herein by reference and attached in the Appendix of priority application U.S. 63/220,312.

### SUMMARY

Generally, example catheters presented herein have an elongated shaft defining a longitudinal axis of the catheter and a circular region at a distal end of an elongated shaft that is generally traverse to the longitudinal axis. The circular region can contract in circumference (and therefore diameter) when a pull wire extending through the circular region and shaft is manipulated by a control handle at a proximal end of the shaft. The circular region can have electrodes distributed around the circumference that are suitable for mapping and/or ablation, and preferably suitable for IRE ablation. The example catheters can include several elongated elements (e.g. said pull wire, electrical wire, mechanical support structure, and/or irrigation tube, etc.) extending through a cross-over region near a distal end of the shaft in which some or all of those elongated elements are angled in relation to the longitudinal axis. The cross-over region can be bounded by an intermediate tube having four lumens and a distal tube having three lumens. One or more of the lumens of the distal tube can be non-coaxial to the lumens of the intermediate tube so that elongated elements extending between the non-coaxial lumens are therefore angled in relation to the longitudinal axis in the cross-over region. The example catheters can include features to provide structural support when forces are applied to contract the circular region such as a support member having a preferable bending direction, polymer tubing segments positioned to inhibit bending stress on electrodes, position of navigation sensors to inhibit bending stress on said sensors and electrodes, and a distal assembly at a distal end of the circular region, etc. A tubular body of the circular region can measure approximately 8 French. The overall construction of some of the example catheters can result in an ability for greater contraction of the circular region compared to existing catheters having similar dimensions.

A first example catheter includes an elongated shaft extended along a longitudinal axis, an intermediate section extended along the longitudinal axis distal from the elongated shaft, and a distal section extending distally from the intermediate section. The intermediate section can include an intermediate tube having a first plurality of lumens therethrough. The distal section can have a generally straight region extended along the longitudinal axis distal from the intermediate section and a circular main region distal from the generally straight region and generally orthogonal to the longitudinal axis. The distal section can include a distal tube having a second plurality of lumens therethrough.

The first example catheter can include a cross-over region between a distal end of the intermediate tube and a proximal end of the distal tube. The first example catheter can include a support member affixed within in the intermediate section within a first lumen of the first plurality of lumens, extended through the cross-over region so that the support member is angled in relation to the longitudinal axis, and extended through the distal section within a second lumen of the second plurality of lumens that is non-coaxial to the first lumen.

The first plurality of lumens, and thereby the intermediate tube, can have four or more lumens. The second plurality of lumens, and thereby the distal tube, can have three or more lumens. Preferably, the first plurality of lumens can include exactly four lumens, and the second plurality of lumens can include exactly three lumens.

The first example catheter can further include a first pull wire extended through the intermediate section within a third lumen of the first plurality of lumens non-coaxial to the first lumen, extended through the cross-over region so that the first pull wire is angled in relation to the longitudinal axis, extended through the second lumen, and affixed within the distal section approximate a distal end of the circular main region. The circular main region can be configured to resize in diameter in response to manipulation of the first pull wire.

The first example catheter can further include a second pull wire extended through and anchored within a fourth lumen of the first plurality of lumens of the intermediate section. The fourth lumen can be non-coaxial to the first lumen and the third lumen. The intermediate section can be configured to deflect from the longitudinal axis in response to manipulation of the second pull wire.

The first example catheter can further include a navigation sensor assembly having inductive coils positioned within the circular main region and a first plurality of wires extending proximally from the inductive coils. The first plurality of wires can extend through a fifth lumen of the second plurality of lumens non-coaxial to the first lumen, through the cross-over region so that the first plurality of wires are angled in relation to the longitudinal axis, and through the first lumen.

The first example catheter can further include electrodes distributed around the circular main region and configured to provide electrical energy to ablate intracardiac tissue using irreversible electroporation.

The first example catheter can further include a second plurality of wires extended proximally from the electrodes through a sixth lumen of the second plurality of lumens, extended through the cross-over region so that the second plurality of wires are angled in relation to the longitudinal axis, and extended through a seventh lumen of the first plurality of lumens non-coaxial to the sixth lumen.

The first example catheter can further include a navigation sensor assembly having inductive coils positioned within the circular main region so that each of the inductive coils is respectively encircled by one of the electrodes.

The electrodes can each have an outer diameter of about 8 French, a length of about 3 millimeters, and an effective surface area of about 21 square millimeters. The electrodes can be separated by an edge-to-edge distance of about 4 millimeters. The electrodes can be configured to withstand 900 Volts between adjacent electrodes and configured to withstand 1800 Volts between alternate electrodes.

The first example catheter can further include polymeric tube segments each having a length of about 7 millimeters, each positioned over the support member, and each positioned centrally under a respective electrode to thereby provide preferential bending locations along the circular main region between the electrodes for at least a portion of the electrodes.

The first example catheter can further include a distal advanced current localization sensor affixed over the generally straight region of the distal section and a proximal advanced current localization sensor affixed over the intermediate section.

The support member can be configured, within the circular main region, to be more flexible in a radial direction that is orthogonal to the longitudinal axis compared to flexibility in the direction of the longitudinal axis. The support member can have an approximately rectangular cross sectional shape approximate a distal end of the distal section. The support member can have an approximately semicircular cross sectional shape within the intermediate section. The rectangular cross sectional shape can have a height and width such that the width is at least twice the height, the width being measured approximately parallel the longitudinal axis and the height being measured approximately orthogonal to the longitudinal axis.

The first example catheter can further include an atraumatic polymer dome at a distal end of the distal section and a knotted cord including ultra high weight molecular polymer disposed within the distal section and anchoring the polymer dome to the distal section.

The first example catheter can further include a tubular sleeve having a length measuring approximately 7 millimeters, circumscribing a distal portion of the distal section approximate a distal end of the distal section, and inhibiting flexion of the distal portion.

A second example catheter an include an elongated shaft extended along a longitudinal axis, a distal section distal of the elongated shaft, a support member extended through the distal section, and a pull wire extended through the distal section. The distal section can include a generally straight region extended along the longitudinal axis distal from the elongated shaft and a circular main region distal from the generally straight region and generally orthogonal to the longitudinal axis. The support member can be configured to be more flexible in a radial direction that is orthogonal to the longitudinal axis compared to flexibility in the direction of the longitudinal axis. The pull wire can be affixed approximate a distal end of the distal section and can be manipulated to radially contract the support member and thereby radially contract the circular main region of the distal section.

The support member can have an approximately rectangular cross sectional shape approximate a distal end of the distal section and comprising an approximately semicircular cross sectional shape approximate a proximal end of the distal section. The rectangular cross sectional shape can have a height and width such that the width is at least twice the height, the width being measured approximately parallel the longitudinal axis and the height being measured approximately orthogonal to the longitudinal axis.

The second example catheter can further include electrodes distributed around the circular main region and configured to provide electrical energy to ablate intracardiac tissue using irreversible electroporation. The electrodes can each have an outer diameter of about 8 French, a length of about 3 millimeters, and an effective surface area of about 21 square millimeters. The electrodes can be separated by an edge-to-edge distance of about 4 millimeters.

The second example catheter can further include polymeric tube segments each having a length of about 7 millimeters, each positioned over the support member, and each positioned centrally under a respective electrode to thereby provide preferential bending locations along the circular main region between the electrodes.

The second example catheter can further include a navigation sensor assembly including inductive coils positioned within the circular main region so that each of the inductive coils is respectively encircled by one of the electrodes.

The second example catheter can further include a distal assembly at a distal end of the distal section distal of the electrodes. The distal assembly can include a distal region of the support member, a distal region of the pull wire, a steel ferrule joining the distal region of the pull wire to the distal region of the support member, a knotted cord including ultra high weight molecular polymer, a distal region of a distal tube (in which the distal region of the support member, the distal region of the pull wire, the steel ferrule, and the knotted cord are disposed), a polymer reinforcing tube circumscribing the distal region of the distal tube, and an atraumatic polymeric tip engaged with the knotted cord and extending distally from a distal end of the distal tube.

The second example catheter can further include an intermediate section distal of the elongated shaft and proximal of the distal section. The intermediate section can include an intermediate tube having a first plurality of lumens therethrough. The support member and the pull wire can be disposed in separate lumens of the first plurality of lumens. The second example catheter can further include a cross-over section distal of the intermediate section and proximal of the distal section. The distal section can include a distal tube having a second plurality of lumens. The support member and the pull wire can be disposed together in a single lumen of the second plurality of lumens.

The second example catheter can further include a navigation sensor assembly including inductive coils positioned within the circular main region and wires extending proximally from the coils. The wires can be disposed in the distal tube in a separate lumen from the single lumen in which the support member and pull wire are disposed. The wires can be disposed in the intermediate tube together in the lumen of the first plurality of lumens in which the support member is disposed.

A third example catheter can include an elongated shaft extended along a longitudinal axis, an intermediate section extended along the longitudinal axis distal from the elongated shaft, a distal section distal of the intermediate section, elongated structures extending through the distal section and the intermediate section, and a cross-over region between the distal section and the intermediate section in which at least one of the elongated structures is angled in relation to the longitudinal axis. The intermediate section can include an intermediate tube having four lumens therethrough. The distal section can include a generally straight region extended along the longitudinal axis distal from the intermediate section and a circular main region distal from the generally straight region and generally orthogonal to the longitudinal axis. The distal section can include a distal tube having three lumens therethrough. The elongated structures can extend through the three lumens of the distal tube and into at least some of the four lumens of the intermediate tube. The cross-over region can be between a distal end of the intermediate tube and a proximal end of the distal tube.

The elongated structures can include a support structure forming the circular main region into a generally circular shape, a pull wire affixed to the support structure, electrode wires, and navigation sensor wires. The support structure, and thereby the circular main region, can be configured to resize in diameter in response to manipulation of the pull wire. Any combination of the elongated structures extending through the cross-over region can be angled in relation to the longitudinal axis.

The third example catheter can further include electrodes distributed around the circular main region and configured to provide electrical energy to ablate intracardiac tissue using irreversible electroporation.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, systems, and methods by way of example only, not by way of limitation.
Figure 1 is an illustration of a profile view of an example catheter according at aspects of the present invention.
Figure 2A is an illustration of a profile view of an intermediate section, cross-over section, and distal section of the example catheter according at aspects of the present invention.
Figure 2B is an illustration of a distal end view of the distal section of the example catheter according at aspects of the present invention.
Figure 2C is an illustration of a distal end of the distal section according at aspects of the present invention.
Figure 3A is an illustration of a profile view of the cross-over section and adjacent portions of the intermediate section and distal section according at aspects of the present invention.
Figure 3B is an illustration of an orthogonal cross-sectional view of the catheter portion illustrated in Figure 3A.
Figure 3C is a cross-sectional view of the intermediate section as indicated in Figure 3A.
Figure 3D is a cross-sectional view of the distal section as indicated in Figure 3A.
Figure 4 is an isometric, cut-away view of the cross-over section and adjacent portions of the intermediate section and distal section according at aspects of the present invention.
Figure 5A is an exploded view of an assembly including a support member, pull wire, and ferrule according at aspects of the present invention.
Figures 5B through 5D are cross-sectional views of the support member as indicated in Figure 5A.
Figure 6A is an assembled view of the assembly illustrated in Figure 5A.
Figure 6B is a zoomed view of a distal portion of the assembly illustrated in Figure 6A.
Figure 7 is a cross-sectional view of the distal section elongated to a linear shape according at aspects of the present invention.
Figure 8A is an illustration of a knotted cord of a distal end assembly of the catheter according at aspects of the present invention.
Figure 8B is a zoomed view of the distal end of the catheter according at aspects of the present invention.

### DETAILED DESCRIPTION

Documents incorporated by reference herein are to be considered an integral part of the application except that, to the extent that any terms are defined in these incorporated documents in a manner that conflicts with definitions made explicitly or implicitly in the present specification, only the definitions in the present specification should be considered.

Although example embodiments of the disclosed technology are explained in detail herein, it is to be understood that other embodiments are contemplated. Accordingly, it is not intended that the disclosed technology be limited in its scope to the details of construction and arrangement of components set forth in the following description or illustrated in the drawings. The disclosed technology is capable of other embodiments and of being practiced or carried out in various ways. Features of embodiments disclosed herein, including those disclosed in the attached Appendix of priority application U.S. 63/220,312, can be combined as understood by a person skilled in the pertinent art according to the teachings herein.

As used herein, the terms "about" or "approximately" for any numerical values or ranges indicate a suitable dimensional tolerance that allows the part or collection of components to function for its intended purpose as described herein. More specifically, "about" or "approximately" can refer to the range of values ±20% of the recited value, e.g. "about 90%" can refer to the range of values from 71% to 99%.

As discussed herein, the term "ablate" or "ablation", as it relates to the devices and corresponding systems of this disclosure, refers to components and structural features of configured to reduce or prevent the generation of erratic cardiac signals. Non-thermal ablation includes use of irreversible electroporation (IRE) to cause cell death, referred throughout this disclosure interchangeably as pulsed electric field (PEF) and pulsed field ablation (PFA). Thermal ablation includes use of extreme temperature to cause cell death and includes RF ablation. Ablating or ablation as it relates to the devices and corresponding systems of this disclosure is used throughout this disclosure in reference to ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" as it generally relates to known methods, devices, and systems includes various forms of bodily tissue ablation as understood by a person skilled in the pertinent art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the terms "biphasic pulse" and "monophasic pulse" refer to respective electrical signals. "Biphasic pulse" refers to an electrical signal having a positive-voltage phase pulse (referred to herein as "positive phase") and a negative-voltage phase pulse (referred to herein as "negative phase"). "Monophasic pulse" refers to an electrical signal having only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Additionally, or alternatively, the system can include a capacitor or other protective component. Where voltage amplitude of the biphasic and/or monophasic pulse is described herein, it is understood that the expressed voltage amplitude is an absolute value of the approximate peak amplitude of each of the positive-voltage phase and/or the negative-voltage phase. Each phase of the biphasic and monophasic pulse preferably has a square shape having an essentially constant voltage amplitude during a majority of the phase duration. Phases of the biphasic pulse are separated in time by an interphase delay. The interphase delay duration is preferably less than or approximately equal to the duration of a phase of the biphasic pulse. The interphase delay duration is more preferably about 25% of the duration of the phase of the biphasic pulse.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The illustrations of the present disclosure depict aspects of an example catheter 10 having a circular end effector with electrodes thereon that can be used for mapping and/or ablation of tissue and can be particularly useful for diagnosis and/or treatment of cardiac arrythmias. The depicted example catheter 10 can be modified to include compatible features of other catheters known to a person skilled in the pertinent art, including compatible features of the catheters disclosed in the attached Appendix of priority application U.S. 63/220,312. Likewise, catheters known to a person skilled in the pertinent art, including those described in the attached Appendix of priority application U.S. 63/220,312, can be modified to include compatible features of the example catheter 10. Further, the example catheter 10, and variations thereof, can be used to perform compatible treatments known to a person skilled in the pertinent art including compatible treatments described in the attached Appendix of priority application U.S. 63/220,312.

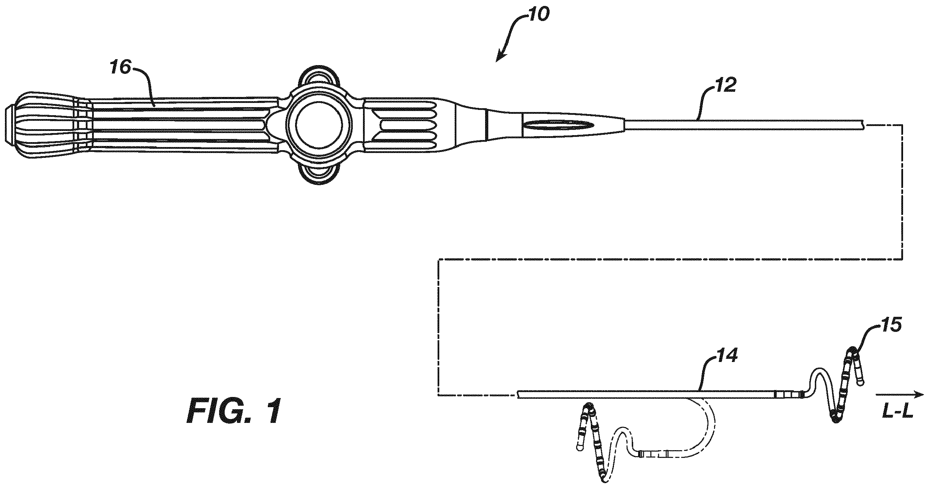

Figure 1 is an illustration of a profile view of the example catheter 10. The catheter 10 includes a distal section 15 having a circular region that is generally traverse to a longitudinal axis L-L defined by an elongated shaft 12 of the catheter 10. The catheter 10 includes an intermediate section 14 extended along the longitudinal axis L-L distal from the elongated shaft 12. The intermediate section 14 can deflect from the longitudinal axis L-L in response to manipulation of a handle 16 at a proximal end of the catheter 10. As illustrated, the intermediate section 14 can be capable of bending approximately 180°.

In an example treatment, a suitable guiding sheath is inserted into the patient with its distal end positioned at a desired treatment location. An example of a suitable guiding sheath for use with the example catheter 10 is the Vizigo^{™} Braiding Guiding Sheath, commercially available from Biosense Webster, Inc. (California, USA). The distal end of the sheath is guided into one of the atria. As the catheter 10 is fed through the guiding sheath, the distal section 15 is straightened to fit through the sheath. Once the distal end of the catheter is positioned at the desired treatment location, the guiding sheath is pulled proximally, allowing the intermediate section 14 and the distal section 15 to extend outside the sheath, and the distal section 15 is free to move to its circular shape. The distal section is then inserted into a pulmonary vein or other tubular region (such as the coronary sinus, superior vena cava, or inferior vena cava) so that the outer circumference of the generally circular main region 39 is in contact with a circumference inside the tubular region. Preferably at least about 50%, more preferably at least about 70%, and still more preferably at least about 80% of the circumference of the generally circular main region is in contact with a circumference inside the tubular region.

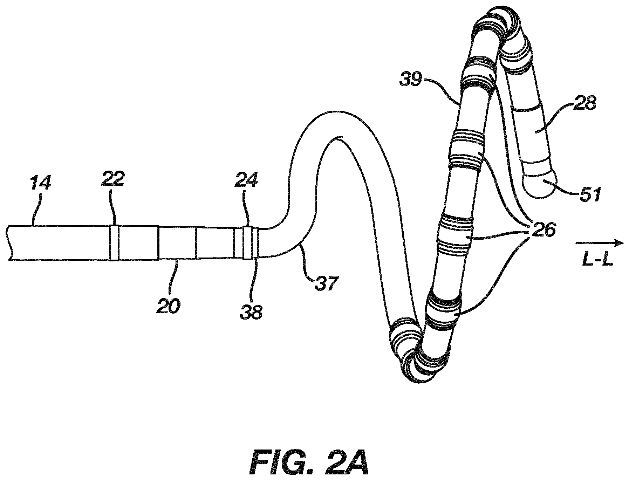

Figure 2A is an illustration of a profile view of a distal portion of the catheter 10 including a distal portion of the intermediate section 14, the distal section 15, and a cross-over tube 20 positioned between the intermediate section 14 and the distal section 15. The distal section 15 includes a generally straight region 38 distal from the intermediate section 14. The generally straight region 38 is aligned with the intermediate section 14 and thereby the longitudinal axis L-L when the intermediate section 14 is aligned with the catheter shaft 12. The distal section 15 includes an elbow 37 between the generally straight region 38 and the generally circular main region 39 of the distal section 15. The circular region 39 is distal from the straight region 38 and generally orthogonal to the longitudinal axis L-L. The circular region 39 is preferably generally perpendicular to the catheter body 12. The circular region 39 can form a flat circle or can be slightly helical, as shown in Figure 2A.

The circular region 39 includes electrodes 26 distributed around its circumference. The electrodes 26 can be configured for mapping and/or ablation. During an example treatment in which the electrodes 26 are used to map electrical activity, the circular arrangement of the electrodes 26 permits measurement of the electrical activity at a circumference of the tubular bodily structure so that ectopic beats between the electrodes can be identified. The size of the generally circular main region 39 can permit measurement of electrical activity along a diameter of a pulmonary vein or other tubular structure of or near the heart when the circular main region has a diameter generally corresponding to that of a pulmonary vein or the coronary sinus. In an example treatment in which the electrodes 26 are used to perform ablation, the circular arrangement of the electrodes 26 facilitates formation of a circular, or ring-shaped lesion to interrupt electrical activity through the circumference of the tubular bodily structure, thereby electrically isolating the tubular structure from tissue on the opposite side of the ring-shaped lesion.

Mapping and/or ablation can be performed using the Carto^{®} system produced by Biosense Webster or other suitable systems as understood by a person skilled in the pertinent art including compatible systems described in the attached Appendix of priority application U.S. 63/220,312. The catheter 10 can be used to perform mapping and/or ablation as part of a compatible treatments including compatible methods of treatment described in the attached Appendix of priority application U.S. 63/220,312.

The intermediate section 14 and the straight region 38 of the distal section 15 respectively include a proximal ring electrode 22 and a distal ring electrode 24 that can be used to determine location and orientation of the intermediate section 14 and straight region 38 of the distal section 15. The ring electrodes 22, 24 can be tracked using impedance measurements at the ring electrodes for instance by using the Advanced Catheter Location (ACL) system, produced by Biosense Webster, which is described in U.S. Patent No. 8,456,182 incorporated herein by reference and attached in the Appendix of priority application U.S. 63/220,312.

The distal end of the distal section 15 is sealed closed with an atraumatic dome 51 of polyurethane glue or the like. A polymer support tube 28 positioned distal of the electrodes 26 provides structural support of a distal portion of the distal section 15 and inhibits flexion of the distal portion.

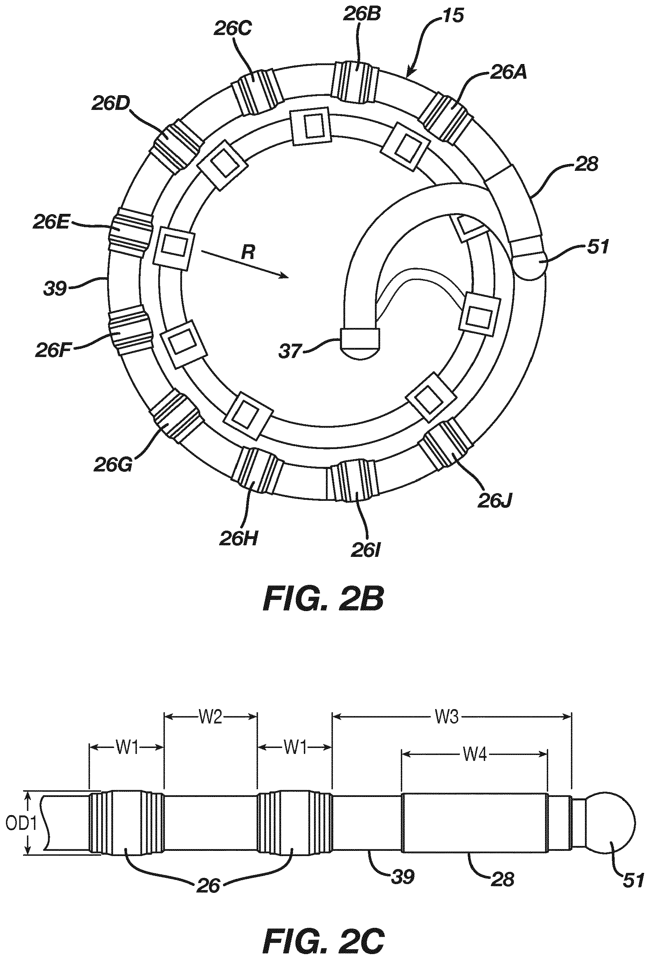

Figure 2B is an illustration of a distal end view of the distal section 15 of the example catheter 10. The generally circular main region 39 can curve in a clockwise direction or a counterclockwise direction. The circular region 39 can contract in a radial direction R. When uncontracted, the circular region 39 can have an outer diameter D1 preferably ranging from about 25 mm to about 35 mm. The circular region 39 can have an outer diameter D2 when contracted preferably ranging from about 15 mm to about 25 mm. The circular region 39 can have a pull wire extending therethrough that can be manipulated by the control handle 16 to cause the circular region 39 to contract.

The catheter 10 is preferably configured to provide IRE ablation voltage pulses from the electrodes 26A-J. IRE is a predominantly non-thermal process, which causes an increase of the tissue temperature by, at most, a few degrees for a few milliseconds. It thus differs from RF (radio frequency) ablation, which raises the tissue temperature by between 20 and 70°C and destroys cells through heating. IRE can utilize monophasic pulses or biphasic pulses. Biphasic pulses are preferred to avoid muscle contraction from a DC voltage. The IRE pulses, alone or in combination with RF ablation, can be generated and applied in various treatments such as described in U.S. Patent Pub. No. 2021/0169550, U.S. Patent Pub. No. 2021/0169567, U.S. Patent Pub. No. 2021/0169568, U.S. Patent App. No. 62/949,999 (Attorney Docket No. BIO6206USPSP1), U.S. Patent Pub. No. 2021/0161592, U.S. Patent App. No. 16/731,238 (Attorney Docket No. BIO6208USNP1), U.S. Patent App. No. 16/710,062 (Attorney Docket No. BIO6209USNP1), and U.S. Patent Pub. No. 2021/0186604 incorporated herein by reference and attached in the Appendix of priority application U.S. 63/220,312. U.S. Patent Application No. 16/989,445 claims priority to U.S. 62/949,999 and is published as U.S. Patent Pub. No. 2021/0191642 which is incorporated herein by reference. U.S. Patent Application No. 16/731,238 is published as U.S. Patent Pub. No. 2021/0196372 which is incorporated herein by reference. Application No. 16/710,062 is published as U.S. Patent Pub. No. 2021/0177503 which is incorporated herein by reference.

In one example treatment, voltage pulses can be applied in a triplet sequence to perform IRE ablation. In a first triplet of the triplet sequence, a biphasic pulse can be applied between a first pair of adjacent electrodes 26A, 26B, next a biphasic pulse of similar amplitude can be applied between a second pair of adjacent electrodes 26B, 26C, and next a biphasic pulse of about twice the amplitude of the previous biphasic pulses can be applied between alternate electrodes 26A, 26C from the previous two pairs of adjacent electrodes. The triplet sequence can continue with a second triplet that includes two adjacent electrodes 26B, 26C from the first triplet and a new adjacent electrode 26D. The second triplet can follow a similar pattern of the first triplet with a biphasic pulse between a first pair of adjacent electrodes 26B, 26C, next a biphasic pulse of similar amplitude between a second pair of adjacent electrodes 26C, 26D, and next a biphasic pulse of about twice the amplitude between alternate electrodes 26B, 26D. The triplet sequence can continue with a third triplet with the next three electrodes 26C, 26D, 26E, a fourth triplet with the next three electrodes 26D, 26E, 26F, a fifth triplet with the next three electrodes 26E, 26F, 26G, a sixth triplet with the next three electrodes 26F, 26G, 26H, a seventh triplet with the next three electrodes 26G, 26H, 261, and an eighth triplet with the next three electrodes 26H, 261, 26J. The triplet pattern can repeat, starting again at the first triplet. The catheter 10 is preferably configured to withstand biphasic pulses between adjacent electrodes having an amplitude of about 900 Volts and biphasic pulses between alternate electrodes having an amplitude of about 1,800 Volts.

Figure 2C is an illustration of a distal end of circular region 39 of the distal section 15 in a straightened configuration. The electrodes 26 preferably have an outer diameter OD1 of about 8 French. The electrodes 26 preferably have a length W1 of about 3 millimeters (mm). The electrodes 26 each preferably have an effective surface area of about 21 mm² each. The electrodes 26 are preferably separated by an edge-to-edge distance W2 of about 4 mm. The distal most electrode 26A can be positioned a length W3 measuring about 10 mm from a distal end of a tubular body of the circular region 39. The polymer support tube 28 preferably has a length W4 of about 7 mm.

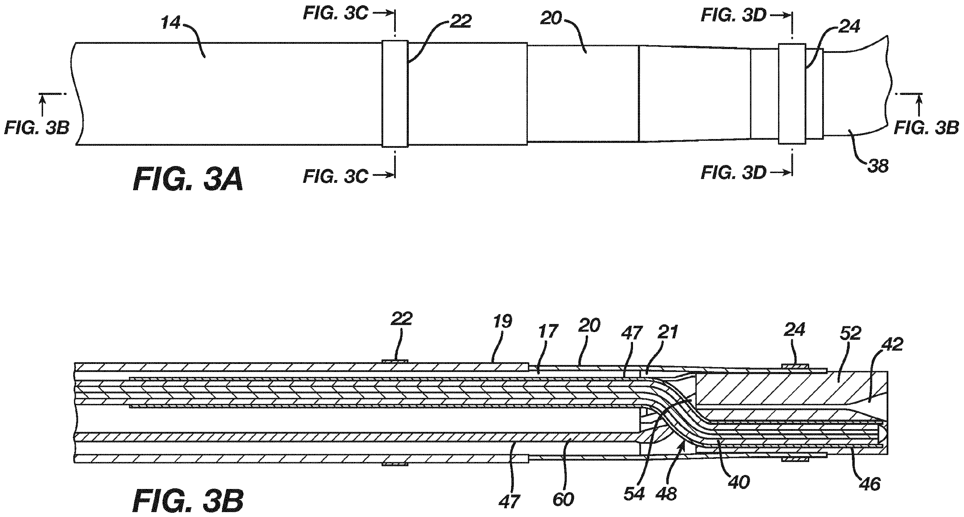

Figure 3A is an illustration of a profile view of the cross-over tube 20 and adjacent portions of the intermediate section 14 and distal section 15.

Figure 3B is an orthogonal cross-sectional view of the catheter portion illustrated in Figure 3A cut in a plane that includes the longitudinal axis L-L. The cross-sectional view of Figure 3BC is indicated in Figure 3A cut in a plane orthogonal to the page and viewed upward to the toward the top of Figure 3A.

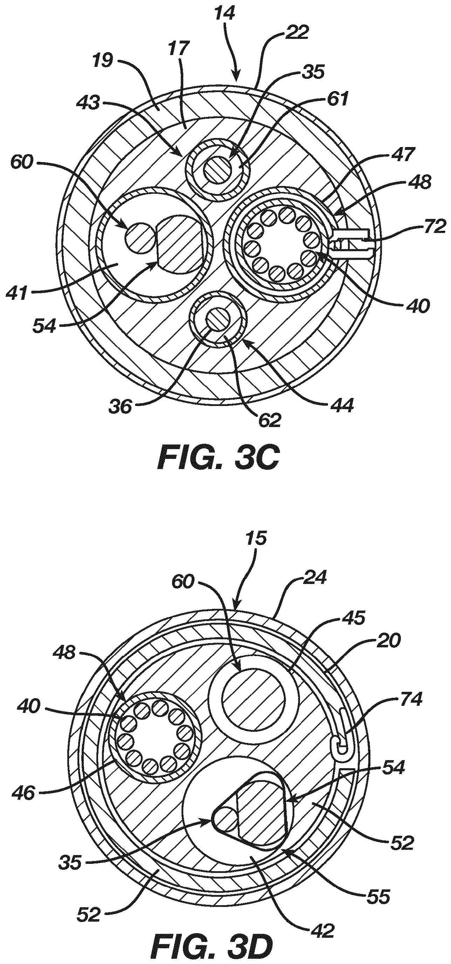

Figure 3C is a cross-sectional view of the intermediate section 14 orthogonal to the longitudinal axis L-L as indicated in Figure 3A in a plane orthogonal to the page and viewed looking distally, toward the right of Figure 3A.

Figure 3D is a cross-sectional view of the distal section 15 as indicated in Figure 3A in a plane orthogonal to the page and viewed looking distally, to toward the right of Figure 3A.

Referring collectively to Figures 3A through 3D, the intermediate section 14 includes an inner, intermediate tube 17 having four lumens 41, 43, 44, 47 therethrough, and the distal section 15 includes a distal tube 52 having three lumens 42, 45, 46 therethrough. The intermediate tube 17 and distal tube 52 can be modified to include alternative numbers of lumens as understood by a person skilled in the pertinent art according to the teachings herein. A cross-over region 21 extends between a distal end 34 of the intermediate tube 17 and a proximal end 32 of the distal tube 52 (see Figure 3B). The cross-over region 21 is surrounded by the cross-over tube 20. The cross-over tube 20 has a single lumen.

The example catheter 10 can include several elongated elements extending through the cross-over region 21. The lumens 42, 45, 46 of the distal tube 52 are non-coaxial to the lumens 41, 43, 44, 47 of the intermediate tube 17. As a result, elongated elements extending between the non-coaxial lumens are angled in relation to the longitudinal axis L-L in the cross-over region 21. Further, the lumens 42, 45, 46 of the distal tube 52 are non-coaxial to each other, and the lumens 41, 43, 44, 47 of the intermediate tube 17 are non-coaxial to each other.

The catheter 10 includes a support member 54 having a proximal end affixed within a first lumen 41, extending distally at an angle to the longitudinal axis L-L through the cross-over region 21, and extending distally into a second lumen 42, where the first lumen 41 is in the intermediate tube 17 and the second lumen 42 is in the distal tube 52. The first lumen 41 and second lumen 42 are non-coaxial to each other, resulting in the angled trajectory of the support member 54 through the cross-over region 21.

The catheter 10 includes a first pull wire/contraction wire 35 extending from the handle 16, through the shaft 12, through a third lumen 43 within the intermediate tube 17, through the cross-over region 21 at an angle to the longitudinal axis L-L, and through the second lumen 42 of the distal tube 52. The third lumen 43 is non-coaxial with the second lumen 42 which causes the contraction wire 35 to be angled within the cross-over region 21. The contraction wire 35 is affixed within the distal section 15 near a distal end of the circular region 39. Manipulation of the handle 16 can cause tension in the contraction wire 35 to cause the circular region 39 to contract in diameter as illustrated in Figure 2B. Within the second lumen 42, the catheter 10 can include a braid 55 surrounding the support member 54 and contraction wire 35 to inhibit the contraction wire 35 from tearing through the distal tube 52 (see Figure 3D). Within the third lumen, the catheter 10 can include a compression coil 61 circumscribing the contraction wire 35 (see Figure 3C).

The catheter 10 includes a second pull wire/deflection wire 36 extending from the control handle 16, through the shaft, and through at least a portion of a fourth lumen 44 of the intermediate tube 17 (see Figure 3C). A distal end of the deflection wire 36 is anchored within the fourth lumen 44. The fourth lumen 44 is non-coaxial to the lumens 42, 45, 46 of the distal tube 52. The intermediate section 14 is configured to deflect from the longitudinal axis L-L in response to manipulation of the deflection wire 36 by the control handle 16. Within the fourth lumen 44, the catheter 10 can include a compression coil 62 circumscribing the deflection wire 36.

The compression coils 61, 62 can be made of any suitable metal, e.g., stainless steel. The compression coils 61, 62 can be tightly wound to provide flexibility, i.e., bending, but to resist compression. The inner diameter of the compression coils 61, 62 is preferably sized slightly larger than the diameter of the associated pull wires 35, 36. For example, when a pull wire 35, 36 has a diameter of about 0.007 inches (about 0.18 mm), the respective compression coil 61, 62 preferably has an inner diameter of about 0.008 inches (about 0.20 mm). A Teflon^{®} coating on each pull wire 35, 36 allows them to slide freely within the compression coils 61, 62. The outer surface of the compression coils 61, 62 can be covered by a flexible, non-conductive tubular member to prevent contact between the compression coils and other components, such as lead wires and cables, etc. Each non-conductive tubular member can be made of polyimide tubing.

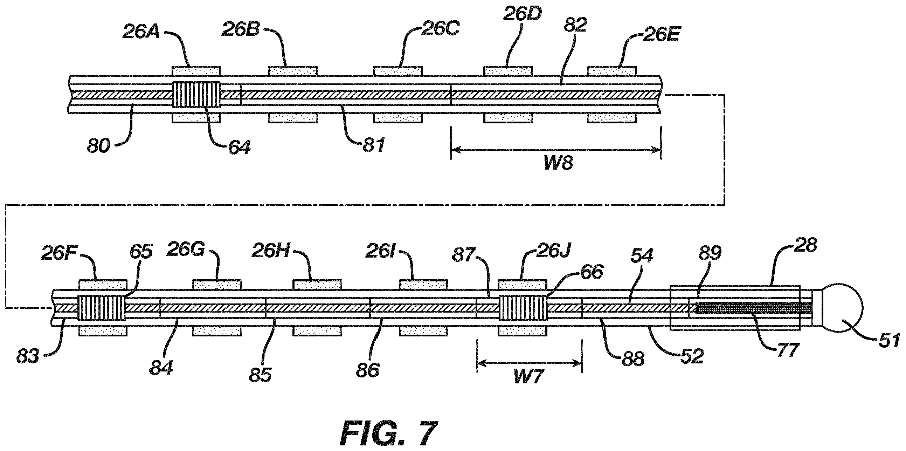

The catheter 10 can include a navigation sensor assembly 60 having inductive coils 64, 65, 66 (see Figure 7) in the circular region 39. The navigation sensor assembly 60 can include conductors (e.g. wires, cables, printed traces, etc.) extending from an electrical connector at the handle 16 (not illustrated), through the shaft 12, through the first lumen 41 in the intermediate tube 17, through the cross-over region 21 at an angle to the longitudinal axis L-L, and through a fifth lumen 45 in the distal tube 52. The fifth lumen 45 is non-coaxial to the first lumen 41 which cause the conductors of the navigation sensor assembly 60 to extend at an angle to the longitudinal axis L-L through the cross-over region 21. Details of the cross-sections of the navigation sensor assembly 60 are omitted for the sake of simplifying the illustrations.

The catheter 10 can include lead wires 40 extending proximally from electrodes 26 on the circular region 39, through a sixth lumen 46 in the distal tube 52, through the cross-over region 21 at an angle to the longitudinal axis L-L, through a seventh lumen 47 through the intermediate tube 17, through the shaft 12, through the control handle 16, and terminate at their proximal end in a connector (not shown) which is connected to an appropriate system configured to receive electrical signals for mapping and/or transmit energy for ablation. The lead wires 40 can be attached to the electrodes 26 of the circular region 39 by any compatible conventional technique. The sixth lumen 46 is non-coaxial to the seventh lumen 47 so that the lead wires 40 are angled in relation to the longitudinal axis L-L through the cross-over region 21. The lead wires 40 can be individually insulated and bundled within an insulating sleeve 48.

The catheter 10 can include a lead wire 72 connected to the proximal ring electrode 22 over the intermediate section 14 (Figure 3C). The catheter 10 can include a lead wire 74 connected to the distal ring electrode 24 over the distal section 15 (Figure 3D).

The catheter 10 can include an outer tube 19 circumscribing the intermediate tube 17 and configured to provide structural stability to the intermediate section 14. The outer tube 19 can extend proximally over the shaft 12 to provide a contiguous outer surface from the shaft 12 to the intermediate section 14.

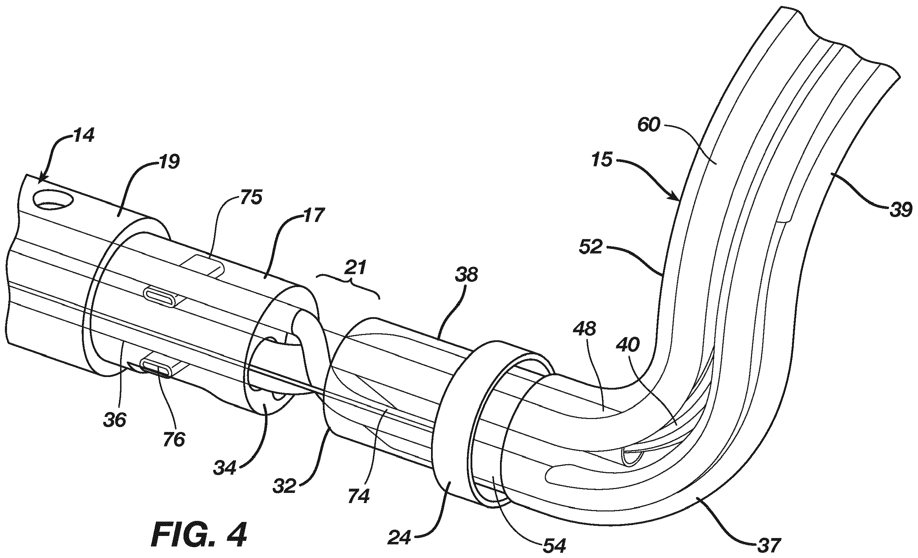

Figure 4 is an isometric view of the cross-over region 21 and adjacent portions of the intermediate section 14 and distal section 15. For the sake of illustration, the cross-over tube 20 is omitted and the outer tube 19, intermediate tube 17, and distal tube 52 are drawn as transparent. Anchor bars 75, 76 for the pull wires 35, 36 are illustrated in the intermediate tube 17.

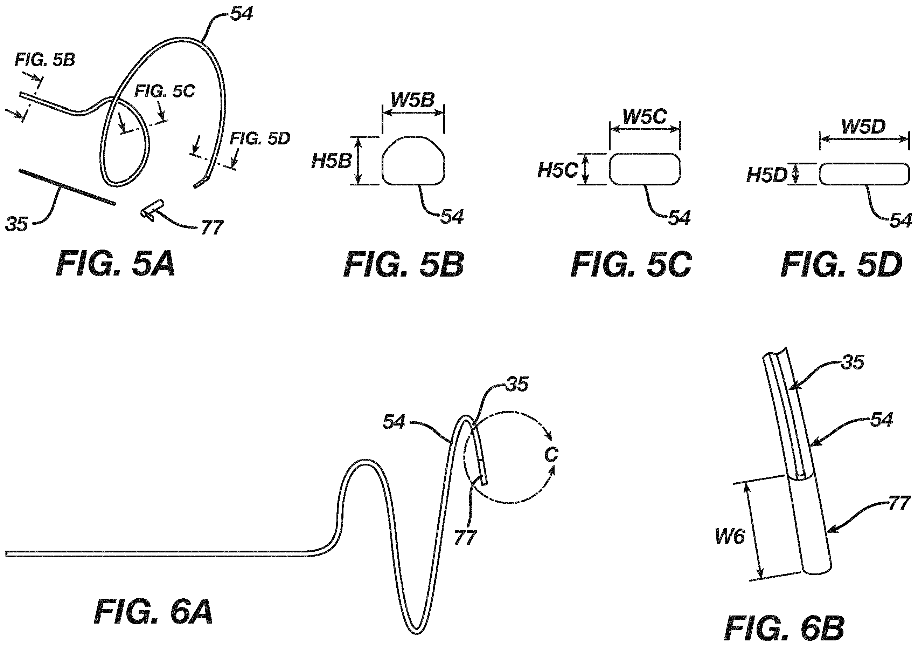

Figure 5A is an exploded view of an assembly including the support member 54, contraction wire 35, and a steel ferrule 77.

Figures 5B through 5D are cross-sectional views of the support member 54 as indicated in Figure 5A. As illustrated in Figure 5B, the support member 54 has an approximately semicircular cross-sectional shape near the proximal end of the support member 54. The support member 54 can have an approximately semicircular shape within the intermediate section 14. The semicircular shape has a width W5B approximately equal to or slightly greater than its height H5B. As illustrated in Figures 5C and 5D, the support member 54 becomes progressive flatter toward the distal end of the support member 54 the width W5C, W5D increases as the height H5C, H5D decreases. Near the distal end of the support member 54, the width W5D can be at least twice the height H5D.

The support member 54 is oriented so that in the circular region 39, the width is approximately aligned with the longitudinal axis L-L and the height is aligned with the radial direction R (see Figure 2B). Oriented as such, the support member 54 is configured to bend preferentially in the radial direction R and resist bending in the direction of the longitudinal axis L-L. The support member 54 is therefore more flexible in the radial direction R compared to flexibility in the longitudinal axis L-L.

Figure 6A is an assembled view of the assembly illustrated in Figure 5A. The contraction wire 35 is affixed to a distal end of the support member 54 by a ferrule 77 preferably of steel or other suitable materials. As illustrated, the contraction wire 35 is positioned on a surface of the support member 54 that faces radially inward. When pulled, the contraction wire 35 contracts causing the support member 54 and therefore the circular region 39 to radially contract. The braid 55 (see Figure 3D) bundles the contraction wire 35 and support member 54 together to help the contraction wire 35 contract the support member 54.

Figure 6B is a zoomed view of a distal portion of the assembly illustrated in Figure 6A. The ferrule 77 has a length W6 of preferably from about 2 mm to about 3 mm.

Figure 7 is a cross-sectional illustration of the distal section 15 of the catheter 10 elongated to a linear shape. The drawing is simplified with certain features, such as wires 40 to the electrodes 26, conductors to the navigation sensor assembly 60, braid 55 around the contraction wire 35 and support member 54 omitted, and distal tube 52 omitted solely for the sake of illustration.

Within the circular region 39 of the catheter 10, the navigation sensor 60 includes three single axis sensors (SASs) 64, 65, 66. Each SAS 64, 65, 66 can include one or more inductive coils. The coils can be wound around the support member 54. Each of the coils can have conductors (e.g. wires) extending proximally therefrom. The coils and wires can be configured as presented in U.S. Patent Pub. No. 2020/0015703 incorporated herein by reference and attached in the Appendix of priority application U.S. 63/220,312.

As illustrated, each of the SASs 64, 65, 66 is encircled by a respective electrode 26A, 26E, 261. These electrodes 26A, 26E, 261 thereby each provide structural support to inhibit bending of the respective SAS 64, 65, 66.

A distal sensor 66, a middle sensor 65, and a proximal sensor 64 are spaced so that the navigation sensor 60 is capable of determining position of the distal section 15 in three dimensions when the distal section has the generally circular shape. Preferably the sensors 64, 65, 66 are approximately 120° from each other when the distal section 15 has the generally circular shape; however, the sensors 64, 65, 66 may deviate from this spacing as the circular shape is resized and/or to accommodate placement under the electrodes 26A, 26E, 261. When the electrode length W1 is 3 mm, the length between electrodes W2 is 4 mm, and the distal section 15 is linear as illustrated in FIG. 7, a center of the distal sensor 66 is preferably separated from a center of the middle sensor 65 by about 28 mm and the center of the middle sensor 65 is separated from a center of the proximal sensor 64 by about 35mm.

The distal section 15 further includes polymer tube segments 80, 81, 82, 83, 84, 85, 86, 87, 88, 89 positioned over the support member 54. Most of the tube segments 83, 84, 85, 86, 87 are approximately centered under a respective electrode 26A, 26B, 26C, 26D, 26E such that ends of these tube segments meet between these electrodes, further promoting flexing of the circular region 39 between electrodes 26A-F and relieving stress to these electrodes when the circular region 39 contracts. These tube segments 83, 84, 85, 86, 87 preferably have a length W7 that is approximately equal to electrode length W1 plus length between electrodes W2 (see Figure 2C). For instance, when the electrode length W1 is 3 mm and the length between electrodes W2 is 4 mm, the tube segment length W7 is preferably about 7 mm. The distal section 15 can include one or more additional tube segments 88, 89 positioned distal of the distalmost electrode 26A to provide structural support at the distal end of the circular region 39. The distal section 15 can include tube segments 81, 82 that extend under two respective electrodes 26F, 26G, 26H, 261. These longer tube segments 81, 82 are preferably positioned in a proximal direction in relation to the previously described tube segments 83, 84, 85, 86, 87, 88, 89 and preferably have a length of about twice the length W7 of the shorter tube segments 83, 84, 85, 86, 87. These longer tube segments 81, 82 can have ends that abut adjacent tube segments between electrodes to promote flexing of the circular region 39 between electrodes where the ends abut. The distal section 15 can further include a proximal tube segment 80 extending under the proximal electrode 26J and having an end between the proximal electrode 26J and adjacent electrode 261. The tube segments 80, 81, 82, 83, 84, 85, 86, 87, 88, 89 preferably include polymeric material.

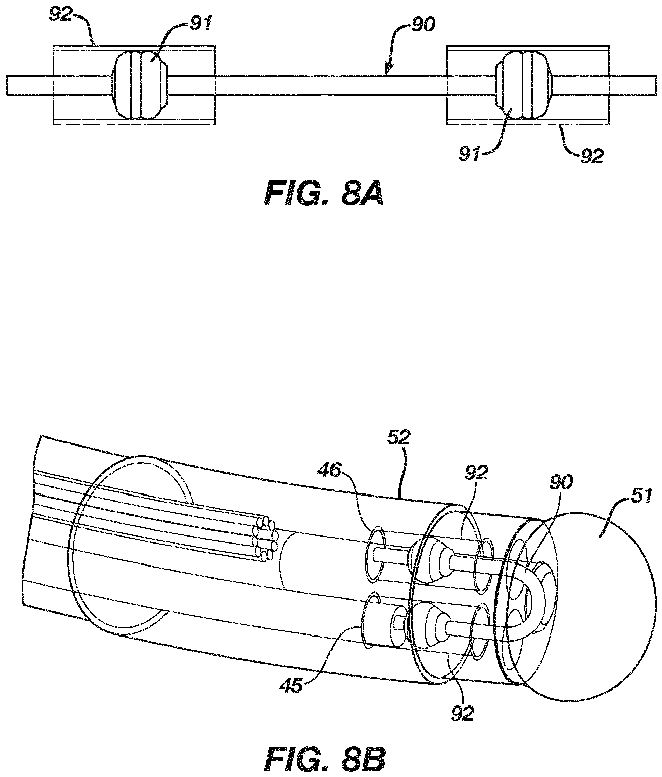

Figure 8A is an illustration of a knotted cord 90 of a distal end assembly of the catheter 10. The knotted cord 90 preferably includes an ultra high weight molecular polymer (e.g. Vectran^{®}) and/or high strength braided fiber. The cord 90 includes one or more knots 91 positioned within one or more polymer (e.g. polymide) tubes 92.

Figure 8B is a zoomed view of the distal end of the catheter 10. The cord 90 is positioned within the distal tube 52 and the atraumatic distal tip 51 of the distal section 15 can be formed by flowing polymeric material such as polyurethane glue, cyanoacrylate, epoxy, or the like into the distal end of the distal section 15. The flowed material can solidify around the knots 91 in the cord 90, conforming to the shape of the cord 90 and engaging the knots 91. The knotted cord 90 can anchor the flowed material into the distal end of the distal section 15 to reduce the likelihood that the atraumatic distal tip 51 becomes unseated during manipulation of the catheter 10.

## Claims

1. A catheter comprising:
an elongated shaft extended along a longitudinal axis;
an intermediate section extended along the longitudinal axis distal from the elongated shaft and comprising an intermediate tube comprising a first plurality of lumens therethrough;
a distal section comprising a generally straight region extended along the longitudinal axis distal from the intermediate section, a circular main region distal from the generally straight region and generally orthogonal to the longitudinal axis, and a distal tube comprising a second plurality of lumens therethrough;
a cross-over region between a distal end of the intermediate tube and a proximal end of the distal tube; and
a support member affixed within in the intermediate section within a first lumen of the first plurality of lumens, extended through the cross-over region so that the support member is angled in relation to the longitudinal axis, and extended through the distal section within a second lumen of the second plurality of lumens that is non-coaxial to the first lumen.

2. The catheter of claim 1,
wherein the first plurality of lumens, and thereby the intermediate tube, comprises four lumens, and
wherein the second plurality of lumens, and thereby the distal tube, comprises three lumens,
preferably wherein the first plurality of lumens consists of four lumens, and the second plurality of lumens consists of three lumens.

3. The catheter of claim 1, further comprising:
a first pull wire extended through the intermediate section within a third lumen of the first plurality of lumens non-coaxial to the first lumen, extended through the cross-over region so that the first pull wire is angled in relation to the longitudinal axis, extended through the second lumen, and affixed within the distal section approximate a distal end of the circular main region, preferably the circular main region being configured to resize in diameter in response to manipulation of the first pull wire.

4. The catheter of claim 1, further comprising:
a second pull wire extended through and anchored within a fourth lumen of the first plurality of lumens of the intermediate section, the fourth lumen being non-coaxial to the first lumen and the third lumen, the intermediate section being configured to deflect from the longitudinal axis in response to manipulation of the second pull wire.

5. The catheter of claim 1, further comprising:
a navigation sensor assembly comprising inductive coils positioned within the circular main region and comprising a first plurality of wires extending proximally from the inductive coils through a fifth lumen of the second plurality of lumens non-coaxial to the first lumen, extended through the cross-over region so that the first plurality of wires are angled in relation to the longitudinal axis, and extended through the first lumen.

6. The catheter of claim 1, further comprising:
electrodes distributed around the circular main region and configured to provide electrical energy to ablate intracardiac tissue using irreversible electroporation.

7. The catheter of claim 6, further comprising:
a second plurality of wires extended proximally from the electrodes through a sixth lumen of the second plurality of lumens, extended through the cross-over region so that the second plurality of wires are angled in relation to the longitudinal axis, and extended through a seventh lumen of the first plurality of lumens non-coaxial to the sixth lumen.

8. The catheter of claim 6, further comprising:
a navigation sensor assembly comprising inductive coils positioned within the circular main region so that each of the inductive coils is respectively encircled by one of the electrodes.

9. The catheter of claim 6, i) the electrodes each comprising an outer diameter of about 8 French, a length of about 3 millimeters, and an effective surface area of about 21 square millimeters, ii) the electrodes being separated by an edge-to-edge distance of about 4 millimeters, or iii) the electrodes being configured to withstand 900 Volts between adjacent electrodes and configured to withstand 1800 Volts between alternate electrodes.

10. The catheter of claim 6, further comprising:
polymeric tube segments each comprising a length of about 7 millimeters, each positioned over the support member, and each positioned centrally under a respective electrode to thereby provide preferential bending locations along the circular main region between the electrodes for at least a portion of the electrodes.

11. The catheter of claim 1, further comprising:
a distal advanced current localization sensor affixed over the generally straight region of the distal section; and
a proximal advanced current localization sensor affixed over the intermediate section.

12. The catheter of claim 1, the support member configured, within the circular main region, to be more flexible in a radial direction that is orthogonal to the longitudinal axis compared to flexibility in the direction of the longitudinal axis.

13. The catheter of claim 12, the support member comprising an approximately rectangular cross sectional shape approximate a distal end of the distal section and comprising an approximately semicircular cross sectional shape within the intermediate section, the rectangular cross sectional shape preferably comprising a height and width such that the width is at least twice the height, the width being measured approximately parallel the longitudinal axis and the height being measured approximately orthogonal to the longitudinal axis.

14. The catheter of claim 1, further comprising:
an atraumatic polymer dome at a distal end of the distal section; and
a knotted cord comprising ultra high weight molecular polymer, disposed within the distal section, and anchoring the polymer dome to the distal section.

15. The catheter of claim 1, further comprising:
a tubular sleeve having a length measuring approximately 7 millimeters, circumscribing a distal portion of the distal section approximate a distal end of the distal section, and inhibiting flexion of the distal portion.
